# EUROPEAN PATENT SPECIFICATION

(11) **EP 3 125 993 B1**
(45) Date of publication and mention of the grant of the patent: **05.05.2021**
(21) Application number: 15717944.1
(22) Date of filing: 27.03.2015
(51) Int. Cl.: A61N 1/36, A61N 1/04, A61N 1/20, A61N 1/08

(54) **SYSTEM FOR TRANSCRANIAL STIMULATION OF A SUBJECT**
SYSTEM ZUR TRANSKRANIALEN STIMULATION EINER PERSON
SYSTÈME POUR LA STIMULATION TRANSCRÂNIENNE D'UN SUJET

(30) Priority: 03.04.2014 GB 201406050
(43) Date of publication of application: 08.02.2017
(73) Proprietor: Sooma OY, 00510 Helsinki (FI)
(72) Inventor: NEUVONEN, Tuomas, FI-02140 Espoo (FI); VIRTANEN, Jani, FI-01150 Söderkulla (FI)
(74) Representative: Aaltonen, Janne Lari Antero
(86) International application number: PCT/FI2015/050211
(87) International publication number: WO 2015/150625

(56) References cited:
- WO-A2-2009/137683
- CN-A- 102 698 360
- US-A1- 2011 319 975
- US-A1- 2012 209 346
- US-A1- 2013 204 315

## Description

The present disclosure generally relates to transcranial direct current simulation, and more specifically relates to transcranial stimulation of a head region of a subject; the present disclosure is, for example, concerned with systems and methods for transcranial stimulation of head regions of subjects. Moreover, aspects of the disclosure are also directed to software products recorded on machine-readable data storage media, wherein such software products are executable upon computing hardware, to implement the aforesaid methods of the disclosure.

### BACKGROUND

Transcranial direct current simulation (tDCS) is a form of neurostimulation which includes delivering a constant and low current directly to a brain region of a person, namely a subject, through electrodes. tDCS is useful for treating patients with psychiatric and neurological disorders, such as major depressive disorder, schizophrenia, chronic pain and tinnitus, and brain injuries, such as strokes and for enhancing language and mathematical abilities, addressing attention span problem, for enhancing problem solving abilities, for improving memory, and enhancing coordination of body movements.

A known tDCS device includes an anode, a cathode and a battery powered device that is operable to deliver a constant current output. The anode is a positively charged electrode and the cathode is a negatively charged electrode. During treatment, one of these electrodes is optionally placed over a head region of a person and another electrode is placed at another location, such as a neck region or shoulder region of the person; however, both electrodes are optionally connected to the head region of the person. Once the electrodes are placed correctly, a stimulation procedure may be started. The battery powered device includes one or more controls for setting the current signal as well as for adjusting a duration of the stimulation procedure. The constant current output flows from the anode through a skull and brain of the person and thereafter to the cathode, creating an electrical circuit. A current density J of an electrode is defined as J=I/A, where I is electric current flowing across the electrode, and A is area of the electrode.

However, a problem associated with existing tDCS systems is uneven current distribution across electrodes, such that some areas of the electrode have higher current density than others. The problem of uneven distribution may be caused by either improper placement of the electrodes or uneven distribution of conducting gel between the head region and the electrode, and may lead to ineffective medical treatment and/or discomfort to patient. The problem of uneven distribution may be minimized by re-adjusting the electrodes and re-distributing the conducting gel when a patient reports discomfort. However, the problem of uneven distribution goes unnoticed and leads to an ineffective treatment when the patient does not report the same.

US 2013/204315 discloses a device where an electrode is positioned in the head of the user. In one embodiment of the disclosure, each electrode can be used for current steering. The said embodiment enables virtual movement of the electrodes. WO 2009/137683 discloses a cap for transcranial direct current stimulation. The cap includes two or more electrodes associated with an inner surface of the cap.

Therefore, there exists a need for a transcranial stimulation system that verifies the current distribution across the electrodes prior to commencing transcranial stimulation of the subject, and facilitates even distribution of current across the electrodes.

### SUMMARY

The present disclosure seeks to provide a system for transcranial stimulation of a subject. The invention is defined in the claims. As far as methods are mentioned, they are provided for illustrative purposes only and do not form part of the claimed invention.

In one aspect, embodiments of the present disclosure provide a system for transcranial stimulation of a subject, wherein the system includes a control arrangement, an electrode arrangement comprising a plurality of electrodes coupled to at least one electrode drive arrangement, wherein the plurality of electrodes are operable to contact onto skin of the head region of the subject, wherein at least one of the plurality of electrodes includes a plurality of electrode elements which are operable in combination to deliver a current of their corresponding at least one electrode to the head region of the subject, and wherein the at least one electrode drive arrangement is arranged to excite and monitor in operation the electrode elements in an individually controlled and monitored manner, and the at least one electrode drive arrangement is operable substantially to even out current distribution between the plurality of electrode elements of the at least one electrode when in contact onto skin of the head region of the subject, wherein the control arrangement is configured to perform conductivity and/or impedance tests of the plurality of electrodes and the plurality of electrode elements when in contact with skin of the subject, prior to commencing transcranial stimulation of the head region of the subject.

In an embodiment of the present invention, the plurality of electrode elements of the electrodes is disposed in a two-dimensional array configuration when engaging in operation onto the head of the subject.

In an embodiment of the present invention, the plurality of electrode elements of the electrodes is disposed in a one-dimensional array configuration when engaging in operation onto the head of the subject.

In an embodiment of the present invention, the at least one electrode drive arrangement is operable substantially to even out current distribution between the plurality of electrode elements of the at least one electrode when in contact onto skin of the head region of the subject to within a variation of less than 50 %, more preferably less than 20 %, and most preferably less than 10 %.

Aspects of the present disclosure provide a transcranial stimulation system that includes a plurality of electrodes and a power controller, in which each electrode includes a plurality of electrode elements, and the power controller individually controls each electrode element, and measure current densities of each electrode element prior to commencing the transcranial stimulation of a subject. When the current densities of the electrode elements are substantially similar, the power controller indicates to the user that there is an even distribution of current across the electrode elements. When the current densities of the electrode elements are not substantially similar, the power controller indicates the same to the user and may enable the user to even out the current distribution across the electrode elements.

Additional aspects, advantages, features and objects of the present disclosure would be made apparent from the drawings and the detailed description of the illustrative embodiments construed in conjunction with the appended claims that follow.

It will be appreciated that features of the invention are susceptible to being combined in various combinations without departing from the scope of the invention as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the invention is not limited to specific instrumentalities disclosed herein. Moreover, those in the art will understand that the drawings are not to scale. Wherever possible, like elements have been indicated by identical numbers.
Fig. 1 is an illustration of a system for transcranial stimulation of a subject, that is suitable for practicing various implementations of the present disclosure;
Fig. 2 is a detailed illustration of a system for transcranial stimulation of a subject, in accordance with the present disclosure;
Figs. 3a and 3b are illustrations of a head band for performing transcranial stimulation of a subject, in accordance with an embodiment of the present disclosure;
Figs. 4a and 4b are detailed illustrations of an exemplary head band, in accordance with an embodiment of the present disclosure;
Fig. 4c is an illustration of a one-dimensional array of electrode elements formed from conducting stripes of the head band of Fig.4a, in accordance with an embodiment of the present disclosure;
Fig. 4d is a schematic illustration of a two-dimensional array of electrode elements, in accordance with an embodiment of the present disclosure; and
Fig. 5 is an illustration of steps of a method of using the system of Fig. 2 for transcranial stimulation of a subject, in accordance with the present disclosure.

### DETAILED DESCRIPTION OF ILLUSTRATIVE EMBODIMENTS

The following detailed description illustrates embodiments of the disclosure and ways in which it can be implemented. Although the best mode of carrying out the invention has been disclosed, those in the art would recognize that other embodiments for carrying out or practicing the invention are also possible.

In overview, a system for transcranial stimulation of a head region of a subject is provided, wherein the system includes an electrode arrangement comprising a plurality of electrodes coupled to at least one electrode drive arrangement. The plurality of electrodes are operable to contact onto skin of the head region of the subject and at least one electrode includes a plurality of electrode elements which are operable in combination to deliver a current of their corresponding at least one electrode to the head region of the subject. Further, the electrode drive arrangement is arranged to excite and/or monitor the electrode elements in an individually controlled and/or monitored manner.

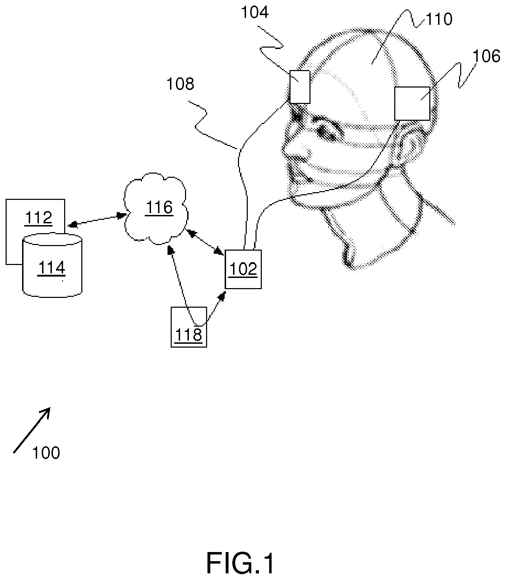

Referring now to the drawings, particularly by their reference numbers, Fig. 1 is an illustration of a system **100** that is suitable for practicing various implementations of the present disclosure. The system **100** includes a power controller **102** and electrical contacts **104** and **106** connected to the power controller **102** through wires **108**, and positioned on a head region **110** of a subject, for example a user. The electrical contact **104** is a positive electrode, *i.e.*, anode **104**, and the electric contact **106** is a negative electrode, *i.e.* cathode **106.** As an example, the anode contact **104** can be connected to a region of skin of a forehead of the subject, and the cathode **106** can be connected to a region of skin in a scalp region of the head region **100** of the subject. The power controller **102** includes an electrode drive arrangement that generates electric signals for driving the anode **104** and cathode **106** to cause transcranial stimulation of the head region **110** of the subject. In an example, the power controller **102** includes a power source for providing a substantially constant low current to the anode **104**, which flows through the skull and brain to the cathode **106**, thereby creating a circuit. Low current includes currents of less than 10 milliAmperes (mA), usually in an order of microamperes (A). Optionally, the substantially constant low current is less than 2 mA.

The power controller **102** further includes a microcontroller (not shown) connected to the power source for enabling an operator and/or the user to set one or more parameters of transcranial stimulation of the head region **110.** In an embodiment, the power controller **102** is operated locally by an operator. In another embodiment, the power controller **102** is operated remotely by a remote operator using an application executing on a remote server system **112**, wherein the server system **112** includes a database **114** and is connected to the power controller **102** via a communication network **116.** Examples of the communication network **116** include, but are not limited to: Internet, Local Area Network (LAN) and Wide Area Network (WAN). In yet another embodiment, the power controller **102** is operated locally by an operator using their mobile wireless communication device **118.** Examples of the mobile wireless communication device **118**, include, but are not limited to, a smart phone, a cell phone, a mobile telephone, a wireless-enabled tablet computer, a wireless-enabled phablet computer, a wearable wireless-enabled computer, and a wireless-enabled wrist-worn computer.

Fig. 1 is merely an example, which should not unduly limit the scope of the claims herein.

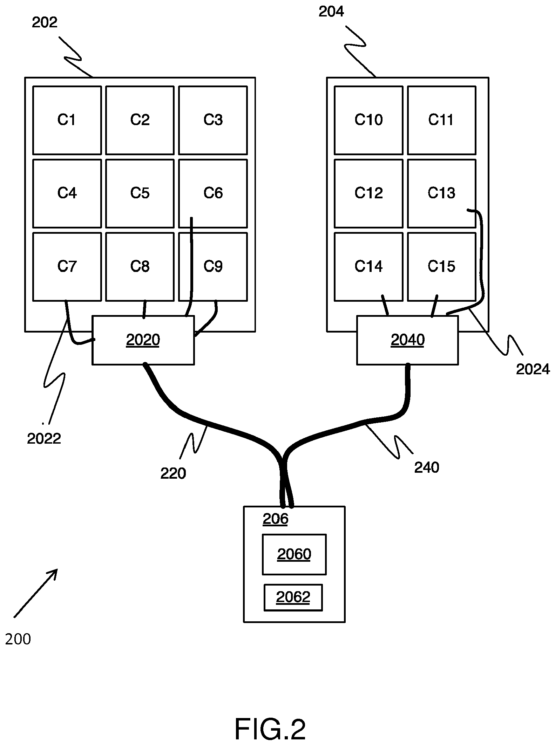

Fig.2 is a detailed illustration of a system **200** for transcranial stimulation of a head region of a subject, in accordance with the present disclosure. The system **200** includes an anode **202**, a cathode **204**, and a power controller **206.** The anode **202**, which is an example of the anode **104**, includes first through ninth electrode elements C₁ till C₉ (hereinafter collectively referred to as anode elements) arranged in a two-dimensional array, and made from a conducting material through which electricity can flow for transcranial stimulation of the head region. Similarly, the cathode **204**, which is an example of the cathode **106**, includes tenth through fifteenth electrode elements C₁₀ till C₁₅ (hereinafter collectively referred to as cathode elements) arranged in a two-dimensional array, and made from a conducting material through which electricity can flow for transcranial stimulation of the head region.

Although, the cathode and anode elements are arranged in a two-dimensional array, it would be obvious to one of ordinary skill in the art, that the anode and cathode elements can be arranged arbitrarily, or in a one-dimensional array, or in a circular array, or in a triangular array depending upon the type of treatment of the subject.

Each anode element is connected separately to a first adapter unit **2020** through one or more wires **2022.** Similarly, each cathode element is connected separately to a second adapter unit **2040** through one or more wires **2024.** The first and second adapter units **2020** and **2040** are configured to switch and control current flows to the anode and cathode elements respectively, and are connected to the power controller **206** (which is an example of the power controller **102**) through wires **220** and **240** respectively. The first and second adapter units **2020** and **2040** are further configured to measure currents flowing through each of the anode and cathode elements. In an embodiment of the present disclosure, the wires **220** and **240** include electricity feed as well as control line such as I₂C connection.

For the first through fifteenth electrode elements C₁ till C₁₅, respective currents flowing across are referred to as first through fifteenth currents I₁ till I₁₅, respective areas are referred to as first through fifteenth areas A₁ till A₁₅, and respective current densities are referred to as first through fifteen current densities J₁ till J₁₅, where Jₙ = Iₙ/Aₙ and n = 1 15, such that J₁ = I₁/A₁, J₂ = I₂/A₂, J₁₅= I₁₅/A₁₅).

The power controller **206** includes a user interface **2060** and a communication interface **2062.** The user interface **2060** includes a display, a touch screen interface, one or more buttons, and voice input/output for enabling the user to control the operation of the system **200.** The communication interface **2062** includes a wired/wireless interface such as Bluetooth, WIFI to communicate and couple with smartphones, computer, and so forth.

Operationally, the first through fifteenth electrode elements C₁ till C₁₅ are verified prior to commencing transcranial stimulation of the head region of the subject. During the verification process, the anode and cathode **202** and **204** are placed on the patient s head at predefined positions. Then, a conducting gel is applied between the head region and the anode and cathode **202** and **204.** The user then switches on the power controller **206** by pressing/touching a power button on the user interface **2060.**

In an embodiment of the present disclosure, as soon as the user presses the power button, the power controller **206** is programmed to send a command to the adapter unit **2040** to switch on the tenth to fifteenth electrode elements (C₁₀ till C₁₅) to receive an electric current, and another command to the adapter unit **2020** to switch on the first electrode element C₁ and keep the second through ninth electrode elements C₂ till C₉ in a switched off state.

In an exemplary embodiment, when the total current to be applied to the head region of the user is 2mA, the power controller 206 provides an electric current of 2/9 mA to the first electrode element C₁, and verifies whether same current is received by the cathode unit **206** by measuring current of the wire **240.** Thereafter, it calculates the first current density J₁ based on the first area A₁ and current I₁ flowing across the first electrode element C₁.

After verifying current I₁ and calculating current density J₁ of the first electrode element C₁, the power controller **206** sends a command to the first adapter unit **2020** to switch on the second electrode element C₂ and switch off the first and third through electrode elements C₁, C₃ till C₉. The power controller **206** then verifies an electric current flowing across the second electrode element C₂ and calculates a second current density J₂ of the second electrode element C₂. In the similar manner, the power controller **206** verifies the currents flowing across the third through ninth electrode elements C₃ till C₉, and calculates corresponding third through ninth current densities J₃ till J₉.

The power controller **206** then analyzes the first through ninth current densities J₁ till J₉ to determine whether the anode elements are properly attached to the head region and are providing sufficient current to the head region of the subject. For example, if the first through ninth current densities J₁ till J₉ are same or substantially same, then the power controller **206** concludes that the anode elements are properly attached to the head region, and may indicate on the user interface **2060**, that the current distribution is even across the anode elements. Alternatively, the power controller **206** concludes that anode elements are not properly attached and may indicate the same on the user interface **2060.** Based on the feedback, the user may rectify the problem and may repeat the verification of the anode elements if needed.

The controller **206** is further programmed to verify the cathode elements in a manner similar to that of the anode elements. After the verification of the anode elements is complete, the controller **206** may be programmed to send a command to the first adapter unit **2020** to switch on the first through ninth electrode elements (C₁ till C₉), and another command to the second adapter unit **2040** to switch on the tenth electrode element C₁₀ and keep the eleventh through fifteenth electrode elements C₁₁ till C₁₅ in a switched off state. The controller **206** may then provide an electric current such as 0.2mA to the anode **202**, and verifies the current flowing across the tenth electrode element C₁₀ to be equal to 0.2mA by measuring current across the wire **240.** Thereafter, the controller **206** measures the tenth current density J₁₀ of the tenth electrode element C₁₀. In the similar manner, the controller **206** verifies the currents flowing across the eleventh through fifteenth electrode elements C₁₁ till C₁₅ and measures corresponding eleventh through fifteenth current densities J₁₁ till J₁₅.

The power controller **206** then analyzes the tenth through fifteenth current densities J₁₀ till J₁₅ to determine whether the cathode elements are properly attached to the head region. For example, if the tenth through fifteenth current densities J₁₀ till J₁₅ are same or substantially same, the power controller **206** concludes that the cathode elements are properly attached to the head region, and may indicate on the user interface **2060**, that the current distribution is even across the cathode elements.

Alternatively, the power controller **206** concludes that the cathode elements are not properly attached and indicate the same on the user interface **2060.** Based on the feedback, the user may rectify the problem and may repeat the verification of the cathode elements if needed. It may be noted that the anode and cathode elements may be verified in different orders with different currents. For example, the cathode elements may be verified before the anode elements.

The first through fifteenth electrode elements (C₁ till C₁₅) may also be verified by testing all permutations of impedance and/or conductivity and/or connectivity and/or current flow between each anode element (C₁ till C₉) with each cathode element (C₁₀ till C₁₅). Also, the impedance between the first through ninth electrode elements (C₁ till C₉) within the anode **202** may be measured by configuring the first electrode element C₁ to be an anode element and the second electrode element C₂ to be as a cathode element. In the similar manner, the impedance between the cathode elements (C₁₀ till C₁₅) may be verified within the cathode **204.**

After the measurement of first through fifteenth current densities J₁ till J₁₅ and verification of connectivity among the first through fifteenth electrode elements C₁ till C₁₅, the controller **206** may configure at least one anode element and at least one cathode element for commencing transcranial stimulation of the head region of the subject.

In an embodiment of the present disclosure, if for some treatment related reasons, all current is desired to be directed via a single anode element such as C₂ and evenly received by all the cathode elements (C₁₀ till C₁₅), then the controller **206** may be configured to switch on the electrode elements C₂, C₁₀ till C₁₅ and switch off the electrode elements C₁, and C₃ till C₉. In an example, when the total current through the anode element C₂ is 2mA and the electrode elements C₁ till C₁₅ are of similar size, i.e. 5mm x 5mm, then the current density J₂ of C₂ is J₂ = 2mA / (5mm x 5mm) and current density of each cathode element (C₁₀ till C₁₅) is J = 2mA / (6 x (5mm x 5mm)).

In another embodiment of the present disclosure, the electrode elements through which the current is delivered to the head region can be changed during the treatment, to introduce possible medical effect. Also, current densities and the amount of current may be varied during the course of the treatment. In an example, for first 5 minutes of the treatment, a current may be configured to flow via electrode elements C₁, C₂, and C₅ and for next 5 minutes, the current may be configured to flow via electrode elements C₁, C₇, C₈, and C₉.

In yet another embodiment of the present disclosure, the first and second adapter units **2020** and **2040** may include multiple constant current sources for providing different currents to the first through fifteenth electrode elements C₁ C₁₅, thereby enabling the power controller **106** to set different current densities for the similar sized electrode elements C₁ ... C₁₅.

Fig. 2 is merely an example, which should not unduly limit the scope of the claims herein.

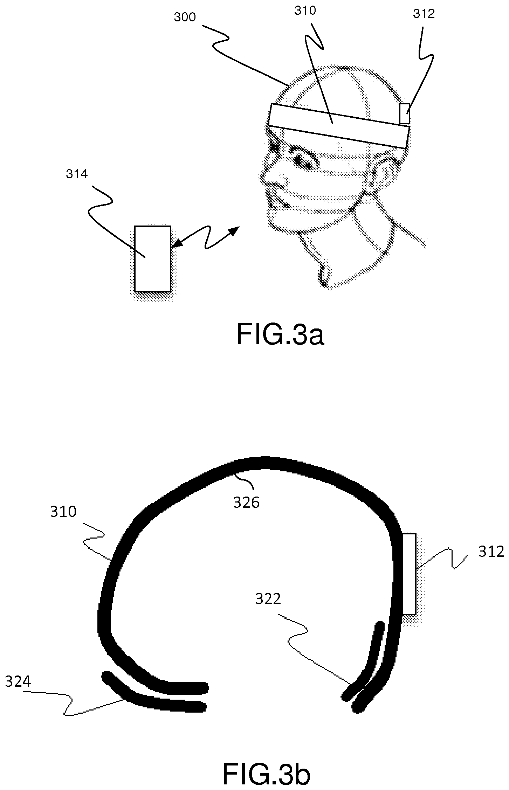

Figs. 3a and 3b are schematic illustrations of a head band **310** for performing transcranial stimulation of a head region **300** of a subject, in accordance with an embodiment of the present disclosure.

The head band **310** includes one or more conducting regions that can be configured as cathode/anode elements for transcranial stimulation of the head region **300.** A control logic **312**, which is an example of the power controller **206**, is attached to the head band **310** and includes mechanical/electrical switching logic to control the switching and operation of the configured cathode/anode elements. The control logic **312** further includes a power source (not shown) to operate the head band **310**, and is configured to communicate with a computing device such as a smart phone **314** of the user.

Further, the head band **310** is configured to be wrapped around the head region **300** of the user by way of attachments **322** and **324.** Example of the attachments **322** and **324** include, but are not limited to Velcro-like loops and Velcro-like hooks respectively. Also, a moisture layer **326** or a coating of nano-materials **326** may be applied to an inner surface of the head band **310** to enable good conductivity between the head band **310** and the head region **300** of the user. *Velcro* is a registered trademark and pertains to a form of fastening product, wherein the product includes first and second pieces of material which are capable of mutually fastening in a releasable manner, by way of micro-hooks of the first piece of material becoming entangled in meshed fibres of the second piece of material. The first and second pieces of material are beneficially manufactured from flexible plastics materials, for example Nylon or similar.

Figs. 3a-3b are merely examples, which should not unduly limit the scope of the claims herein.

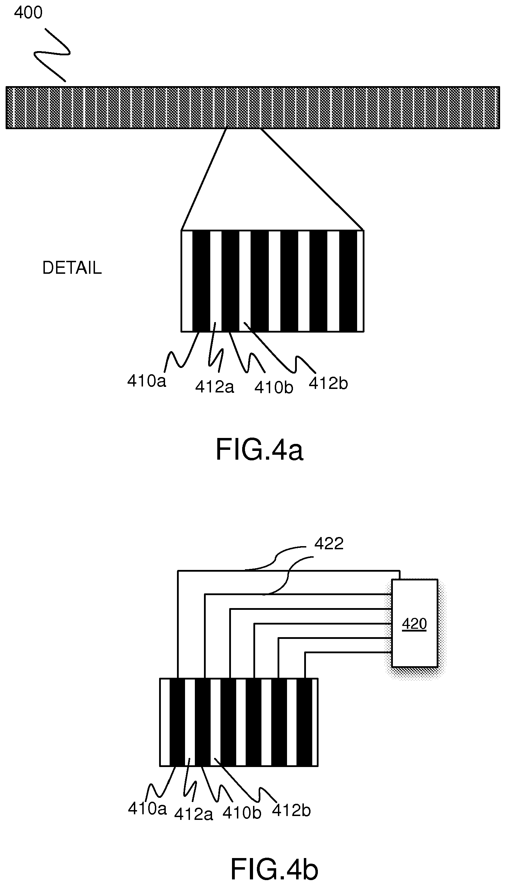

Figs. 4a and 4b are schematic detailed illustrations of an exemplary head band **400**, which is an example of the head band **310**, in accordance with an embodiment of the present disclosure. The head band **400** includes one or more conducting stripes **410a** and **410b** separated by one or more insulating stripes **412a** and **412b**, hereinafter collectively referred to as stripes. In an example, the head band **400** is made from a zebra connector type of material.

The control logic **420**, which is an example of the control logic **312**, is connected to each stripe separately through wires **422** and controls each stripe separately. In some embodiments, the control logic **420** may be connected to two or more stripes together and controls a group of stripes together. The control logic **420** further sets the polarity of each stripe to be positive, negative or floating, thus configuring each stripe either as a cathode element or an anode element. After the stripes are configured as cathode/anode elements, they may be used for transcranial stimulation of the head region of the user.

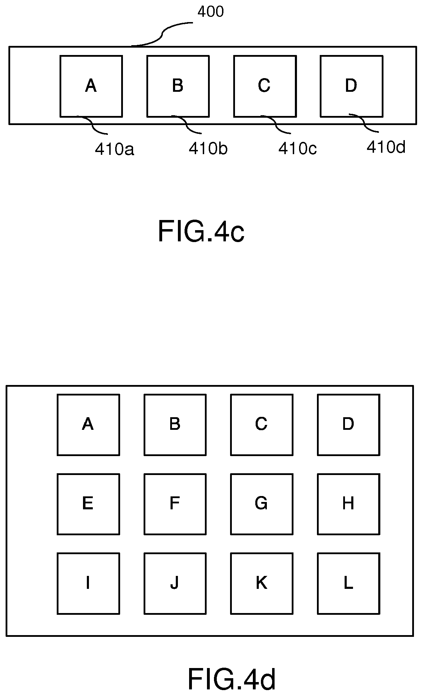

Fig. 4c is a schematic illustration of a one-dimensional array **430** of electrode elements A, B, C and D formed from conducting stripes **410a**, **410b**, **410c** and **410d**, in accordance with an embodiment of the present disclosure. The electrode elements may include anode as well as cathode elements, and may be controlled by the control logic **420.** In an embodiment of the present disclosure, the control logic **420** is configured to measure current and voltage fed via/from the electrode elements A, B, C and D, and measure a resistance and/or impedance between at least one pair of electrode elements based on the measured current and voltages. In an example, the control logic **420** may be configured to measure resistances R_{ab}, R_{ac}, R_{ad}, R_{bc}, R_{bd}, R_{ed} between each possible pair of the electrode elements A, B, C and D. When the resistances R_{ab}, R_{ac}, R_{ad}, R_{bc}, R_{bd}, R_{cd} are substantially similar, the control logic **420** concludes that the electrode elements A, B, C and D may provide substantially equal amount of conductivity to the head region of the subject.

Fig. 4d is a schematic illustration of a two-dimensional array **440** of electrode elements A till L, in accordance with an embodiment of the present disclosure. One or more conducting stripes of the head band **400** may be arranged in a manner so as to form the two-dimensional array **440** of the electrode elements A till L. For the two-dimensional array **440**, resistances and/or impedances between substantially all combinations of the electrode elements A till L may be measured to determine quality of the conductivity to the head region of the subject.

Figs. 4a-4d are merely examples, which should not unduly limit the scope of the claims herein.

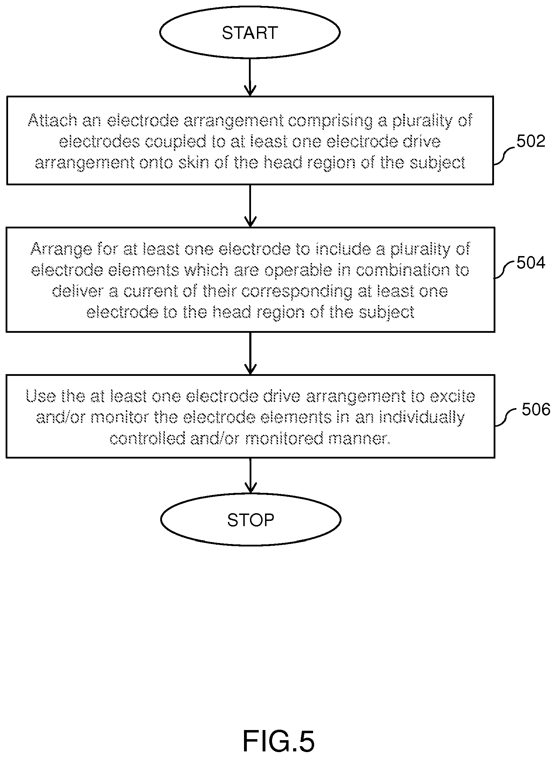

Fig. 5 is an illustration of steps of a method of using a system for transcranial stimulation of a head region of a subject, in accordance with the present disclosure; it will be appreciated that such transcranial stimulation is not limited to methods of treatment of the animal or human body, but can be for other purposes also, for example for relieving stress, for relaxation, for comfort and so forth. The method is depicted as a collection of steps in a logical flow diagram, which represents a sequence of steps that can be implemented in hardware, software, or a combination thereof.

At a step **502**, an electrode arrangement comprising a plurality of electrodes **202** and **204** coupled to at least one electrode drive arrangement **206** is attached onto skin of the head region of the subject.

At a step **504**, the electrode **202** is arranged to include first through ninth electrode elements C₁ till C₉, and another electrode **204** is arranged to include tenth through fifteenth electrode elements C₁₀ till C₁₅, which are operable in combination to deliver a current of their corresponding at least one electrode **202/204** to the head region of the subject.

At a step **506**, at least one electrode drive arrangement **206** is used to excite and/or monitor the first through fifteenth electrode elements C1 till C15 in an individually controlled and/or monitored manner.

It should be noted here that the steps **502** to **506** are only illustrative.

Fig. 5 is merely an example.

Embodiments are not limited to direct current transcranial stimulation. Based on embodiments, conductivity between electrodes can also be verified, for example for transcranial alternative current stimulation (tACS), transcranial alternative current stimulation (tACS), transcranial random noise stimulation (tRNS) and transcutaneous electrical nerve stimulation (TENS).

## Claims

1. A system (100, 200) for transcranial stimulation of a subject, wherein the system (100, 200) includes
- a control arrangement (102, 206, 312, 420);
- an electrode arrangement comprising a plurality of electrodes (202, 204, 400) coupled to at least one electrode drive arrangement, wherein the plurality of electrodes (202, 204) are operable to contact onto skin of the head region (110, 300) of the subject,
**characterised in that** at least one of the plurality of electrodes (202, 204) includes a plurality of electrode elements (C1, C2, C3, C4, C5, C6, C7, C8, C9, C10, C11, C12, C13, C14, C15, 410a, 410b, 412a, 412b) which are operable in combination to deliver a current of their corresponding at least one electrode (202, 204, 400) to the head region (110, 300) of the subject, and wherein the at least one electrode drive arrangement is arranged to excite and monitor in operation the electrode elements (C1, C2, C3, C4, C5, C6, C7, C8, C9, C10, C11, C12, C13, C14, C15, 410a, 410b, 412a, 412b) in an individually controlled and monitored manner and the at least one electrode drive arrangement is operable substantially to even out current distribution between the plurality of electrode elements (C1, C2, C3, C4, C5, C6, C7, C8, C9, C10, C11, C12, C13, C14, C15, 410a, 410b, 412a, 412b) of the at least one electrode (202, 204) when in contact onto skin of the head region (110, 300) of the subject, wherein the control arrangement (102, 206, 312, 420) is configured to perform conductivity and/or impedance tests of the plurality of electrodes (202, 204) and the plurality of electrode elements (C1, C2, C3, C4, C5, C6, C7, C8, C9, C10, C11, C12, C13, C14, C15, 410a, 410b, 412a, 412b) when in contact with skin of the subject, prior to commencing transcranial stimulation of the head region (110, 300) of the subject.

2. A system (100, 200) as claimed in claim 1, wherein the plurality of electrode elements (C1, C2, C3, C4, C5, C6, C7, C8, C9, C10, C11, C12, C13, C14, C15, 410a, 410b, 412a, 412b) of the electrodes (202, 204, 400) are disposed in a two-dimensional array configuration when engaging in operation onto the head of the subject.

3. A system (100, 200) as claimed in claim 1, wherein the plurality of electrode elements (C1, C2, C3, C4, C5, C6, C7, C8, C9, C10, C11, C12, C13, C14, C15, 410a, 410b, 412a, 412b) of the electrodes (202, 204, 400) are disposed in a one-dimensional array configuration (400) when engaging in operation onto the head of the subject.

4. A system (100, 200) as claimed in any of the preceding claims, wherein the at least one electrode drive arrangement is operable substantially to even out current distribution between the plurality of electrode elements (C1, C2, C3, C4, C5, C6, C7, C8, C9, C10, C11, C12, C13, C14, C15, 410a, 410b, 412a, 412b) of the at least one electrode (202, 204, 400) when in contact onto skin of the head region (110, 300) of the subject to within a variation of less than 50 %, more preferably less than 20 %, and most preferably less than 10 %.

## Patentansprüche

1. System (100, 200) zur transkraniellen Stimulation einer Person, wobei das System (100, 200) einschließt
- eine Steueranordnung (102, 206, 312, 420);
- eine Elektrodenanordnung, die eine Vielzahl von Elektroden (202, 204, 400) umfasst, die mit mindestens einer Elektrodenantriebsanordnung gekoppelt sind, wobei die Vielzahl von Elektroden (202, 204) dafür betreibbar sind, um auf der Haut des Kopfbereichs (110, 300) der Person zu kontaktieren,
**dadurch gekennzeichnet, dass** mindestens eine der Vielzahl von Elektroden (202, 204) eine Vielzahl von Elektrodenelementen (C1, C2, C3, C4, C5, C6, C7, C8, C9, C10, C11, C12, C13, C14, C15, 410a, 410b, 412a, 412b) umfasst, die in Kombination dafür betreibbar sind, um einen Strom ihrer entsprechenden mindestens einen Elektrode (202, 204, 400) an den Kopfbereich (110, 300) der Person zu liefern und wobei die mindestens eine Elektrodenantriebsanordnung dafür angeordnet ist, um im Betrieb die Elektrodenelemente (C1, C2, C3, C4, C5, C6, C7, C8, C9, C10, C11, C12, C13, C14, C15, 410a, 410b, 412a, 412b) auf eine individuell gesteuerte und überwachte Weise anzuregen und zu überwachen, und die mindestens eine Elektrodenantriebsanordnung dafür betreibbar ist, um im Wesentlichen eine Stromverteilung zwischen der Vielzahl von Elektrodenelementen (C1, C2, C3, C4, C5, C6, C7, C8, C9, C10, C11, C12, C13, C14, C15, 410a, 410b, 412a, 412b) der mindestens einen Elektrode (202, 204) bei Kontakt mit der Haut des Kopfbereichs (110, 300) der Person auszugleichen, wobei die Steueranordnung (102, 206, 312, 420) dafür konfiguriert ist, um vor Beginnen von transkranieller Stimulation des Kopfbereichs (110, 300) der Person bei Kontakt mit der Haut der Person Leitfähigkeits- und/oder Impedanztests der Vielzahl von Elektroden (202, 204) und der Vielzahl von Elektrodenelementen (C1, C2, C3, C4, C5, C6, C7, C8, C9, C10, C11, C12, C13, C14, C15, 410a, 410b, 412a, 412b) durchzuführen.

2. System (100, 200) nach Anspruch 1, wobei die Vielzahl von Elektrodenelementen (C1, C2, C3, C4, C5, C6, C7, C8, C9, C10, C11, C12, C13, C14, C15, 410a, 410b, 412a, 412b) der Elektroden (202, 204, 400) in einer zweidimensionalen Array-Konfiguration angebracht sind, wenn sie bei Betrieb auf den Kopf der Person eingreifen.

3. System (100, 200) nach Anspruch 1, wobei die mehreren Elektrodenelemente (C1, C2, C3, C4, C5, C6, C7, C8, C9, C10, C11, C12, C13, C14, C15, 410a, 410b, 412a, 412b) der Elektroden (202, 204, 400) in einer eindimensionalen Array-Konfiguration (400) angebracht sind, wenn sie bei Betrieb auf den Kopf der Person eingreifen.

4. System (100, 200) nach einem der vorstehenden Ansprüche, wobei die mindestens eine Elektrodenantriebsanordnung dafür betreibbar ist, um im Wesentlichen die Stromverteilung zwischen der Vielzahl von Elektrodenelementen (C1, C2, C3, C4, C5, C6, C7, C8, C9, C10, C11, C12, C13, C14, C15, 410a, 410b, 412a, 412b) der mindestens einen Elektrode (202, 204, 400) bei Kontakt mit der Haut des Kopfbereichs (110, 300) der Person auf innerhalb einer Variation von weniger als 50 %, mehr bevorzugt weniger als 20 % und am meisten bevorzugt weniger als 10 % auszugleichen.

## Revendications

1. Système (100, 200) pour la stimulation transcrânienne d'un sujet, dans lequel le système (100, 200) inclut
- un agencement de commande (102, 206, 312, 420) ;
- un agencement d'électrodes comprenant une pluralité d'électrodes (202, 204, 400) couplées à au moins un agencement d'excitation d'électrode, dans lequel la pluralité d'électrodes (202, 204) sont opérationnelles pour venir en contact sur la peau de la région de tête (110, 300) du sujet,
**caractérisé en ce qu'**au moins l'une parmi la pluralité d'électrodes (202, 204) inclut une pluralité d'éléments d'électrode (C1, C2, C3, C4, C5, C6, C7, C8, C9, C10, C11, C12, C13, C14, C15, 410a, 410b, 412a, 412b) qui sont opérationnels en combinaison pour acheminer un courant de leur au moins une électrode correspondante (202, 204, 400) vers la région de tête (110, 300) du sujet, et dans lequel l'au moins un agencement d'excitation d'électrode est agencé pour exciter et surveiller en fonctionnement les éléments d'électrode (C1, C2, C3, C4, C5, C6, C7, C8, C9, C10, C11, C12, C13, C14, C15, 410a, 410b, 412a, 412b) d'une manière individuellement commandée et surveillée et l'au moins un agencement d'excitation d'électrode est opérationnel essentiellement pour uniformiser une distribution de courant entre la pluralité d'éléments d'électrode (C1, C2, C3, C4, C5, C6, C7, C8, C9, C10, C11, C12, C13, C14, C15, 410a, 410b, 412a, 412b) de l'au moins une électrode (202, 204) lors d'un contact sur la peau de la région de tête (110, 300) du sujet, dans lequel l'agencement de commande (102, 206, 312, 420) est configuré pour mettre en œuvre des tests de conductivité et/ou d'impédance de la pluralité d'électrodes (202, 204) et de la pluralité d'éléments d'électrode (C1, C2, C3, C4, C5, C6, C7, C8, C9, C10, C11, C12, C13, C14, C15, 410a, 410b, 412a, 412b) lors d'un contact sur la peau du sujet, avant de commencer une stimulation transcrânienne de la région de tête (110, 300) du sujet.

2. Système (100, 200) tel que revendiqué dans la revendication 1, dans lequel la pluralité d'éléments d'électrode (C1, C2, C3, C4, C5, C6, C7, C8, C9, C10, C11, C12, C13, C14, C15, 410a, 410b, 412a, 412b) des électrodes (202, 204, 400) sont disposés dans une configuration de réseau bidimensionnel lors d'une mise en fonctionnement sur la tête du sujet.

3. Système (100, 200) tel que revendiqué dans la revendication 1, dans lequel la pluralité d'éléments d'électrode (C1, C2, C3, C4, C5, C6, C7, C8, C9, C10, C11, C12, C13, C14, C15, 410a, 410b, 412a, 412b) des électrodes (202, 204, 400) sont disposés dans une configuration de réseau unidimensionnel (400) lors d'une mise en fonctionnement sur la tête du sujet.

4. Système (100, 200) tel que revendiqué dans l'une quelconque des revendications précédentes, dans lequel l'au moins un agencement d'excitation d'électrode est opérationnel essentiellement pour uniformiser une distribution de courant entre la pluralité d'éléments d'électrode (C1, C2, C3, C4, C5, C6, C7, C8, C9, C10, C11, C12, C13, C14, C15, 410a, 410b, 412a, 412b) de l'au moins une électrode (202, 204, 400) lors d'un contact sur la peau de la région de tête (110, 300) du sujet avec un écart de variation inférieur à 50 %, plus préférablement inférieur à 20 %, et le plus préférablement inférieur à 10 %.
